# EUROPEAN PATENT SPECIFICATION

(11) **EP 3 060 131 B1**
(45) Date of publication and mention of the grant of the patent: **11.12.2019**
(21) Application number: 14792963.2
(22) Date of filing: 21.10.2014
(51) Int. Cl.: A61B 8/08, A61M 5/19, A61M 5/31, A61B 5/00

(54) **DOUBLE BARREL SYRINGE AND METHOD TO USE SAME FOR PLACEMENT CONFIRMATION AND JOINT SPACE INJECTION**
DOPPELSPRITZENKOERPER UND METHODE ZUR VERWENDUNG DESSELBEN ZUR POSITIONSBESTIMMUNG UND ZUR INJEKTION IN EIN GELENK
SERINGUE DOUBLE CORPS ET SA METHODE D'UTILISATION POUR CONFIRMER LA POISTION ET L'INJECTION DANS L'ESPACE ARTICULAIRE

(30) Priority: 21.10.2013 US 201361893771 P
(43) Date of publication of application: 31.08.2016
(73) Proprietor: Accuro Technologies Inc., Victoria, BC V8R 5J2 (CA)
(72) Inventor: HELLIWELL, James, A., Victoria, BC V8R 3P2 (CA); WILMINK, Michael, Phoenix, Arizona 85008 (US)
(74) Representative: Mewburn Ellis LLP
(86) International application number: PCT/US2014/061608
(87) International publication number: WO 2015/061341

(56) References cited:
- EP-A1- 0 210 160
- EP-A2- 0 312 394
- WO-A1-01/97881
- WO-A1-97/06428
- WO-A1-2007/035621
- WO-A1-2012/139035
- US-A- 3 749 084
- US-A1- 2010 106 097
- US-A1- 2011 118 659

## Description

### BACKGROUND

### Technical Field

The present disclosure generally relates to syringes.

### Description of the Related Art

Patients suffering from acute or chronic pain in their joints typically receive injections in the joint space for relief and therapeutic purposes. These injections are commonly known as intra-articular injections. Intra-articular injections are typically administered by orthopedic surgeons, rheumatologists, and other physicians and health care professionals.

Intra-articular injections typically include therapeutics that assist in pain relief or treatment by administration thereof into the affected areas. In some instances, intra-articular injection therapeutics may be in the form of steroids. Such steroids may have anti-inflammatory properties that can decrease inflammation of the affected joint; provide relief to patients with non-inflammatory arthritis, such as osteoarthritis; or protect joint cartilage. In other instances, the intra-articular injection therapeutics may have properties that improve the lubrication of the joint, reduce pain, or improve range of motion. Still further, in other instances, the intra-articular injection therapeutics may include local anesthetic to provide a temporary analgesic affect.

Administering Intra-articular injections, however, is a complicated procedure, which requires precise positioning. A substantial portion of intra-articular injections are not effectively administered because of the complex human anatomy and precise positioning required to inject the therapeutic into the joint space. This often results in physicians and professionals using expensive, time-consuming, and complex medical imaging tools to properly administer intra-articular injection in the three-dimensional structure of a patient's joint space. Even using medical imaging tools, the physician may miss the precise location, thus failing to deliver effective treatment. New approaches to administration of intro-articular injections are desirable.

US 2010/106097 A1 describes an indicator for a syringe, and particularly to a method and apparatus for testing the compatibility of a fluid before injecting the fluid into a medical device.

WO 2012/139035 A1 describes various devices for providing a single-handed operation of a dual fluid injection of fluid injection/aspiration.

EP 0 312 394 A2 describes devices for immunoassays employing a reusable syringe or vacuum manifold to pass samples, by means of a pressure gradient through a membrane containing an affinity partner for analyte. The devices can also be adapted to direct blood sampling and to automate assays.

US 2011/118659 A1 describes a multi-port syringe assembly that allows fluid to be drawn into or injected out of the assembly. For example, the assembly may include one syringe that is used to aspirate fluid from a patient and another syringe to inject a medical fluid into the patient.

WO 01/97881 A1 describes a method and apparatus for locking of central-vein catheters.

US 3 749 084 A describes a syringe having two or more dispensing chambers therewithin operable to dispense from or fill independent chambers in any desired sequential order.

WO 2007/035621 A1 describes a device for use in intravitreous administration of ocular agents.

EP 0 210 160 A1 describes a plurality of syringe bodies commonly actuatable by pistons and to which a connecting head is attachable, for use in the application of tissue adhesive.

### BRIEF SUMMARY

The invention is as defined in the appended claims

In various implementations, syringes with robust and efficient form factors enable precise placement of a needle tip and sterile administration of intra-articular injections in joint spaces.

A syringe may be summarized as including a hub having an orifice; a first barrel; a second barrel; a first plunger; a second plunger; a valve; and a test indicator. The first and second barrels may have interior surfaces that form a respective first barrel lumen and a second barrel lumen. The first plunger may have a head which is slideably received in the first barrel lumen for movement therein and be in sealing engagement with the interior surface of the first barrel. The second plunger may have a head which is slideably received in the second barrel lumen for movement therein and be in sealing engagement with the interior surface of the second barrel. The valve is operable in a first condition to open a fluidly communicative path between the orifice of the hub and the first barrel lumen and to close a fluidly communicative path between the orifice of the hub and the second barrel lumen. The valve may be operable in a second condition to open the fluidly communicative path between the orifice of the hub and the second barrel lumen and to close the fluidly communicative path between the orifice of the hub and the first barrel lumen. The test indicator may be responsive to at least one characteristic of a bodily fluid, with the test indicator being positioned to be exposed to any bodily fluid drawn into the first barrel lumen and may be visible from an exterior of the first barrel.

A syringe may be summarized as including one or more barrels; a common hub having an orifice; one or more plungers; and at least one valve. The one or more barrels may form a first lumen and a second lumen, where the common hub may be selectively fluidly communicatively coupleable alternatingly to the first lumen and the second lumen. The one or more plungers may have a respective head that may be slideably received in at least one of the first or the second lumens for movement therein to draw bodily fluid into one of the first or the second lumens and to expel contents of the other one of the first or the second lumens. The at least one valve may be operable in a first condition to open a fluidly communicative path between the orifice of the common hub and the first lumen and to close a fluidly communicative path between the orifice of the common hub and the second lumen. The at least one valve may be operable in a second condition to open the fluidly communicative path between the orifice of the common hub and the second lumen and to close the fluidly communicative path between the orifice of the common hub and the first lumen.

The syringe may further include a test indicator responsive to at least one characteristic of a bodily fluid; an injectable fluid; a needle coupleable to the common hub; and a set of instructions. The set of instructions may provide instructions for use of the syringe, wherein the test indicator, the injectable fluid, the needle, and the set of instructions are supplied with the syringe as a kit.

A method may employ a syringe having one or more barrels, a first lumen, a second lumen, a common hub, a needle coupled to the common hub, at least one valve which fluidly communicatively couples the common hub with the first and the second lumens, and at least one test indicator. The method may be summarized as including inserting the needle into bodily tissue, drawing bodily fluid into the first lumen via the needle, checking the at least one test indicator, and in response to a defined visual indication by the test indicator, expelling fluid from the second lumen into the bodily tissue via the needle.

### BRIEF DESCRIPTION OF THE SEVERAL VIEWS OF THE DRAWINGS

Figure 1 is a perspective view of a syringe, according to one embodiment.
Figure 2 is an exploded view of the syringe of Figure 1.
Figure 3 is a cross-sectional view of the syringe of Figure 1 taken along line 3-3.
Figure 4 is a cross-sectional view of a syringe, according to another embodiment.
Figure 5 is a cross-sectional view of a syringe, according to another embodiment.
Figure 6 is an exploded view of a syringe, according to yet another embodiment.
Figure 7 is a cross-sectional view of the syringe of Figure 6.

### DETAILED DESCRIPTION

Unless the context requires otherwise, throughout the specification and claims which follow, the word "comprise" and variations thereof, such as, "comprises" and "comprising" are to be construed in an open, inclusive sense that is as "including, but not limited to."

Reference throughout this specification to "one embodiment" or "an embodiment" means that a particular feature, structure or characteristic described in connection with the embodiment is included in at least one embodiment of the disclosure. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

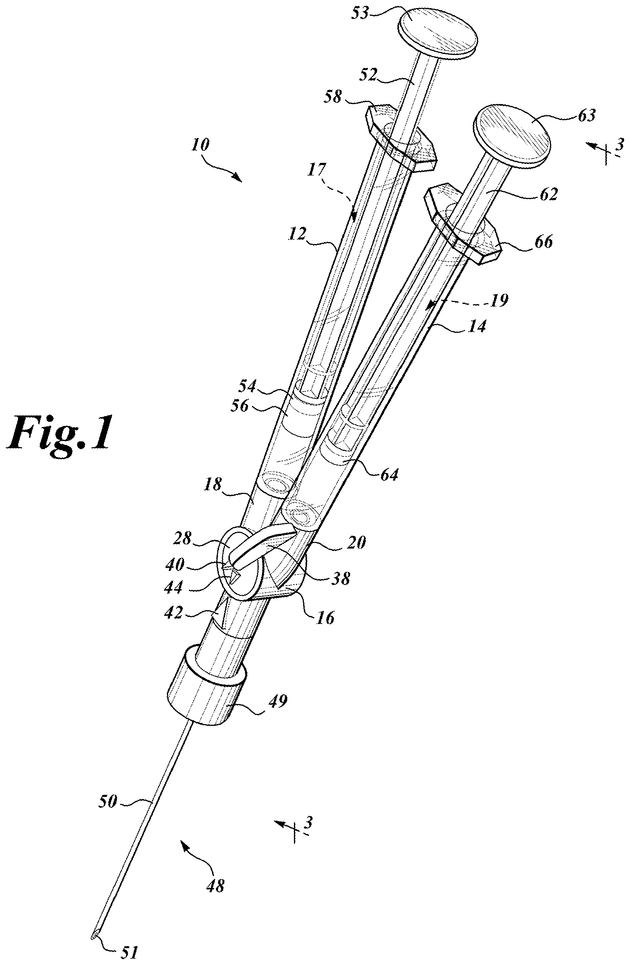

Figures 1 through 3 illustrate a syringe 10, according to one example embodiment. The syringe 10 may, for example, be used to provide intra-articular injections. For example, the syringe 10 may be used to determine where a needle tip is currently positioned within a three-dimensional structure of a joint and to deliver an injectable fluid to a desired location, such as an intra-articular location. The injectable fluid may include a therapeutic agent (*e.g*., an Active Pharmaceutical Agent, such as corticosteroid, hyaluronic acid or a biologic). Alternatively, the injectable fluid may include a diagnostic agent, such as X-ray-contrast preparations, radioactive isotopes, and/or dyes. In some implementations, the injectable fluid may include a combination of a therapeutic agent and a diagnostic agent. The injectable fluid may further include excipients or other pharmaceutically inactive substances formulated with the therapeutic agent and/or diagnostic agents.

The syringe 10 includes a test chamber barrel 12 and an injectable fluid chamber barrel 14. The barrels 12, 14 each have a respective interior space or lumens 17, 19. The test chamber barrel 12 and the injectable fluid chamber barrel 14 may be formed of transparent or translucent materials, such as clear plastic or glass, to allow a user to view the interior of the test chamber and injectable fluid chamber barrels 12, 14. In addition, the test chamber and injectable fluid chamber barrels 12, 14 may include graduation markings to allow the user to view the fluid against the graduation markings to assess the volume of fluid in the respective chamber barrels 12, 14.

An upper end of the test chamber barrel 12 optionally includes a finger flange 58 that extends peripherally around the upper end of the test chamber barrel 12. The finger flange 58 assists a user by providing a gripping surface during use.

An upper end of the injectable fluid chamber barrel 14 optionally includes a finger flange 66 that extends peripherally around the upper end of the injectable fluid chamber barrel 14. The finger flange 66 also assists a user by providing a gripping surface during use.

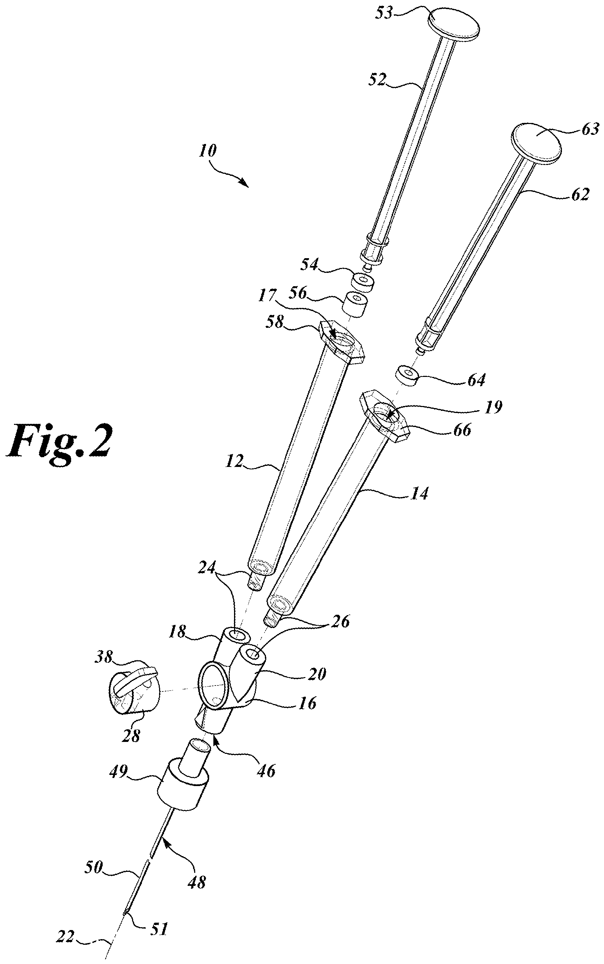

The test chamber barrel 12 and the injectable fluid chamber barrel 14 are coupled to a communal hub 16. The communal hub 16 may, for example, be generally Y-shaped. An upper portion of the Y-shape includes a test chamber adapter 18 and an injectable fluid chamber adapter 20. The test chamber adapter 18 and the injectable fluid chamber adapter 20 are angularly spaced apart from each other about a central reference plane 22 (Figure 2). The angular spacing between the test chamber adapter 18 and the injectable fluid chamber adapter 20 may vary between, for example, 10 degrees to 60 degrees. Coupling adapters 24, 26, for example, in the form of female and male Luer-Lock portions, couple the test chamber and injectable fluid chamber barrels 12, 14 to the communal hub 16. Male couplers, for example, in the form of male Luer-Lock portions are located at respective lower ends of the test chamber barrel 12 and the injectable fluid chamber barrel 14. Female couplers, for example, in the form of female Luer-Lock portions, are located at the respective test chamber adapter 18 and the injectable fluid chamber adapter 20. The male Luer-Lock portions are physically detachably coupleable to corresponding female Luer-Lock portions. In this manner, the test chamber barrel 12 and/or the injectable fluid chamber barrel 14 may each be selectively detachable from the syringe 10.

A central portion of the communal hub 16 is substantially cylindrical and hollow, and includes a valve, for example, a stopcock 28, coupled to the hollow portion of the communal hub 16. The stopcock 28 includes at least a two-way flow system. The two-way flow system includes a main passageway 32, a first connecting passageway 34, and a second connecting passageway 36. The stopcock 28 is operable to selectively fluidly communicatively couple the main passageway 32 to the first or the second connecting passageways 34, 36. By way of example, the stopcock 28 includes a handle 38 which is rotatable such that the stopcock 28 is selectively operable in one of the at least two configurations, namely withdrawal and expulsion. In some implementations, the stopcock 28 is selectively operable in one of three configurations, namely withdrawal, expulsion, and closed (*i.e.,* the main passageway 32 is not fluidly communicatively connected to either one of the first or second connecting passageways 34, 36).

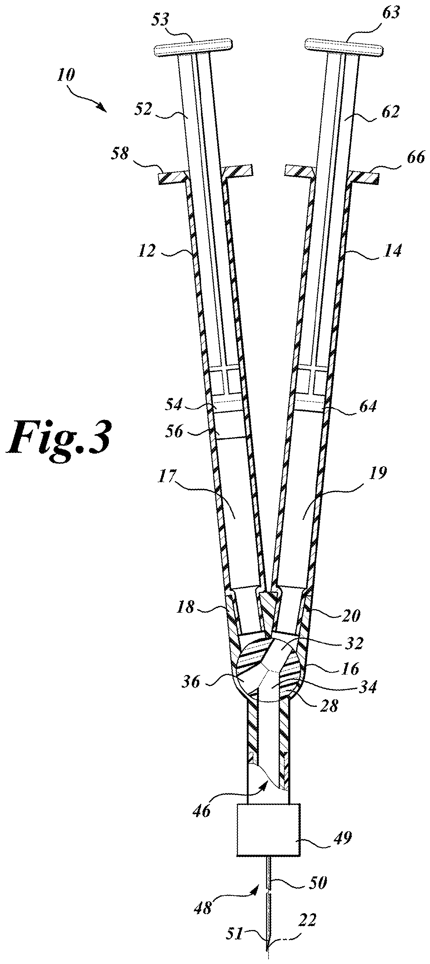

By way of example, rotating the handle 38 counterclockwise such that a withdrawal arrow 40 is aligned with a main arrow 42, fluidly communicatively couples the main passageway 32 with the second connecting passageway 36, allowing a fluid to be drawn to the test chamber barrel 12 from the main passageway 32 in the withdrawal configuration. This counterclockwise rotation of the handle 38 may concurrently fluidly communicatively decouple the main passageway 32 from the first connecting passageway 34. Conversely, rotating the handle 38 in a clockwise direction such that an expulsion arrow 44 is aligned with the main arrow 42, fluidly communicatively couples the main passageway 32 with the first connecting passageway 34, allowing fluid to be expelled from the injectable fluid chamber barrel 14 via the first passageway 34 and the main passageway 32 (Figure 3) in the expulsion configuration. This clockwise rotation of the handle 38 may concurrently fluidly communicatively decouple the main passageway 32 from the second connecting passageway 36.

In the optional closed configuration, the main passageway 32 is not fluidly communicatively coupled with either of the first or the second connecting passageways 34, 36.

While the syringe 10 of Figures 1 through 3 includes a stopcock 28 to selectively provide a fluidly communicative path between the communal hub 16, the test chamber and injectable fluid chamber barrels 12, 14, in other embodiments, the syringe 10 may include other forms of valving mechanisms that are operable to selectively provide one or more fluidly communicative paths.

A lower portion of the communal hub 16 includes an orifice 46 through which a needle 48 is coupled to the communal hub 16. Again, the orifice 46 may be formed by or part of a Luer-Lock connector or coupler. For example, the orifice 46 may be part of a male Luer-Lock portion and the needle 48 may include a female Luer-Lock portion that detachably rotatingly couples with the male Luer-Lock portion. While the embodiment illustrated in Figures 1 through 3 includes a Luer-Lock connection, in other embodiments, the communal hub 16 may include a slip-tip, an eccentric tip, or other types of needle adapters to couple the communal hub 16 to the needle 48.

The needle 48 includes a needle hub 49 and a needle shaft 50. The needle shaft 50 includes a beveled end or point or tip 51, and includes a lumen extending therethrough. The needle 48 is fluidly communicatively coupled to the syringe 10 to withdraw or expel fluid when administering to a patient.

With continued reference to Figures 1 through 3, the syringe 10 includes a first plunger 52 having a head or plunger seal 54 at a lower end of the first plunger 52. The first plunger 52 is partially received in the test chamber lumen 17 of the test chamber barrel 12. The plunger seal 54 sealingly engages with the interior surface of the test chamber barrel 12 which forms the test chamber lumen 17. The plunger seal 54 may be made from rubber or a polymer, such as an elastomer. The plunger 52 is slideably moveable within the test chamber barrel 12 and includes a thumb rest 53.

The syringe 10 further includes a test indicator 56. The test indicator 56 typically provides a visual indication (*e.g*., appearance of line, change of color) when contacted by a defined substance. The test indicator 56 is located in the test chamber lumen 17 so as to be contacted by fluid drawn into the test chamber lumen 17 via operation of the first plunger 52. As illustrated in Figures 1 through 3, the test indicator 56 may take the form of a cylindrical test indicator 56 coupled to the plunger seal 54. Such may, for example, take the form of a strip of material (*e.g*., woven or nonwoven) or substrate, wrapped around an outer perimeter or periphery of the plunger seal 54. Alternatively, the test indicator 56 may take any other of a large variety of forms. For example, the test indicator 56 may take the form of a rectangular shaped strip of a material with one or more substances (*e.g*., reagents) that react in a defined manner (*e.g*., change color) in the presence of defined substances (*e.g*., glucose). The test indicator 56 may additionally or alternatively be coupled to the interior surface of the test chamber barrel 12. Notably, the test indicator 56 should be readily visible from an exterior of the test chamber barrel 12.

The syringe 10 includes a second plunger 62 and a head or plunger seal 64 at a lower end of the plunger 62. The second plunger 62 is partially received in the injectable fluid chamber lumen 19 of the injectable fluid chamber barrel 14. The plunger seal 64 sealingly engages with the interior surface of the injectable fluid chamber barrel 14 which forms the injectable fluid chamber lumen 19. The plunger seal 64 may be made from rubber or a polymer, such as an elastomer. The plunger 62 is slideably moveable within the injectable fluid barrel 14 and includes a thumb rest 63.

The test chamber barrel 12 and the injectable fluid chamber barrel 14 of the syringe 10 operate in tandem to advantageously allow a user to precisely administer intra-articular injections. The user may first rotate the stopcock 28 handle 38 to align the withdrawal arrow 40 with the main arrow 42, fluidly communicatively coupling the main passageway 32 with the second passageway 36 and fluidly communicatively decoupling the main passageway 32 from the first passageway 34.

After bleeding air within the test chamber barrel 12, the user may insert the tip 51 of the needle 48 at the affected joints. The user may then withdraw fluid from the affected joints. For example, distal movement of the first plunger 52 will create a negative pressure in the test chamber barrel 12 to allow the user to withdraw fluid from the patient's joint space. As the fluid contacts the test indicator 56, a chemical reaction between the fluid and a substance (*e.g*., reagent) carried by the test indicator 56 may provide an indication of the presence of fluids which are known to be found in joint spaces. By way of example, joints of a body include bursae, which are filled with synovial fluid. Synovial fluids have a high concentration of glucose that provides essential nutrition to the synovium. Thus, to test such presence, the test indicator 56 may comprise a reagent strip or other strips which use glucose oxidase, hexokinase, or cupric sulfate, for example, to determine the glucose content or presence. The test indicator 56 may indicate presence of glucose colorimetrically, which may be read visually or in some embodiments through a reflectance photometer. The user may continue repositioning and withdrawing the fluid until the test indicator 56 indicates appropriate positioning.

Once the user confirms that the tip 51 of the needle 48 is correctly positioned in the joint space by detecting a change in the test indicator 56, the user may, for example, rotate the stopcock 28 handle 38 to align the expulsion arrow 44 arrow with the main arrow 42, fluidly communicatively coupling the main passageway 32 with the first passageway 34 and fluidly communicatively decoupling the main passageway 32 from the second passageway 36. The user may then precisely apply the injectable fluid (*e.g*., an Active Pharmaceutical Ingredient ("API"), such as corticosteroid, hyaluronic acid or a biologic), by proximally moving the second plunger 62, which will create a positive pressure in the injectable fluid chamber barrel 14, thus expelling the injectable fluid into the joint via the tip 51 of the needle 48.

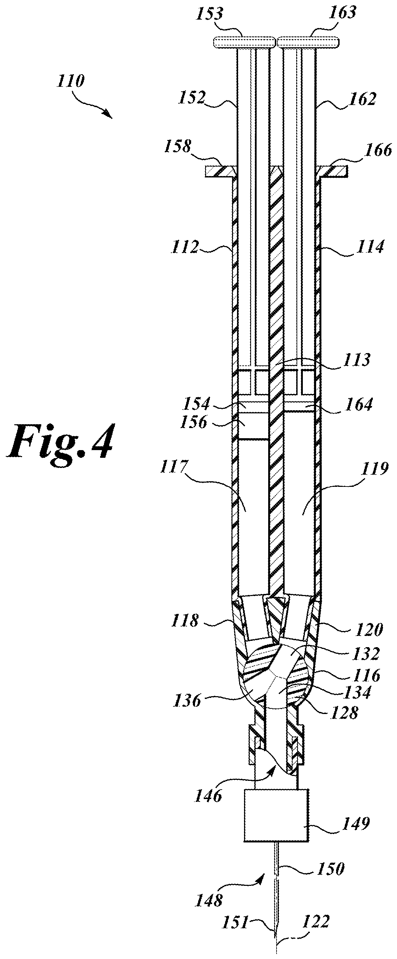

Figure 4 illustrates another example embodiment of a syringe 110. The syringe 110 includes a test chamber barrel 112 and an injectable fluid chamber barrel 114. Each chamber barrel 112, 114 has a respective interior space or lumens 117, 119. The test chamber barrel 112 and the injectable fluid chamber barrel 114 may be integrally formed as a single unit, for example with a web 113 extending therebetween that couples the test chamber barrel 112 to the injectable fluid chamber barrel 114. The syringe 110 may be formed integrally by plastic injection molding. Alternatively, the test chamber barrel 112 and the injectable fluid chamber barrel 114 may be coupled together, for example via adhesive, hook and loop fastener, other fasteners, etc. The test chamber barrel 112 and the injectable fluid chamber barrel 114 may be formed of transparent or translucent materials, such as clear plastic or glass, to allow a user to view fluids in the interior of the test chamber and injectable fluid chamber barrels 112, 114. In addition, the test chamber and injectable fluid chamber barrels 112, 114 may include graduation markings to allow the user to view the fluid against the graduation markings to assess the volume of fluid in the respective chamber barrels 112, 114.

An upper end of the test chamber barrel 112 optionally includes a finger flange 158 that extends peripherally around the upper end of the test chamber barrel 112. The finger flange 158 assists a user by providing a gripping surface during use.

An upper end of the injectable fluid chamber barrel 114 optionally includes a finger flange 166 that extends peripherally around the upper end of the injectable fluid chamber barrel 114. The finger flange 166 also assists a user by providing a gripping surface during use.

The test chamber barrel 112 and the injectable fluid barrel 114 are coupled to a communal hub 116. The communal hub 116 may, for example, be generally Y-shaped. An upper portion of the Y-shape includes a test chamber adapter 118 and an injectable fluid chamber adapter 120. The test chamber adapter 118 and the injectable fluid chamber adapter 120 are angularly spaced apart from each other about a central reference plane 122. The angular spacing between the test chamber adapter 118 and the injectable fluid chamber adapter 120 may vary between, for example, 10 degrees to 60 degrees. By way of example, in the illustrated embodiment of Figure 4, the angular spacing is substantially less than the angular spacing of the embodiment illustrated in Figures 1 through 3. Again, couplers, for example in the form of male Luer-Lock portions are located at respective lower ends of the test chamber barrel 112 and the injectable fluid chamber barrel 114. Complementary couplers, for example female Luer-Lock portions, are located at respective test chamber adapter 118 and the injectable fluid chamber adapter 120. The male Luer-Lock portions are physically detachably coupleable to corresponding female Luer-Lock portions. In this manner, the test chamber barrel 112 and/or the injectable fluid chamber barrel 114 may each be selectively detachable from the syringe 110.

A central portion of the communal hub 116 is substantially cylindrical and hollow, and includes a valve, for example, a stopcock 128, coupled to the hollow portion of the communal hub 116. The stopcock 128 includes at least a two-way flow system. The two-way flow system includes a main passageway 132, a first connecting passageway 134, and a second connecting passageway 136. The stopcock 128 is operable to selectively fluidly communicatively couple the main passageway 132 to the first and second connecting passageways 134, 136. By way of example, the stopcock 128 includes a handle 138 (not shown) which is rotatable such that the stopcock 128 is selectively operable in one of the at least two configurations, namely withdrawal and expulsion. In some implementations, the stopcock 128 is selectively operable in one of three configurations, namely withdrawal, expulsion, and closed (*i.e.,* the main passageway 132 is not fluidly communicatively connected to either one of the first or second connecting passageways 134, 136).

By way of example, rotating the handle 138 counterclockwise such that a withdrawal arrow is aligned with a main arrow, fluidly communicatively couples the main passageway 132 with the second connecting passageway 136, allowing a fluid to be drawn to the test chamber barrel 112 from the main passageway 132, in the withdrawal configuration. This counterclockwise rotation of the handle 138 may concurrently fluidly communicatively decouple the main passageway 132 from the first connecting passageway 134. Conversely, rotating the handle 138 in a clockwise direction such that an expulsion arrow is aligned with the main arrow, fluidly communicatively couples the main passageway 132 with the first connecting passageway 134, allowing fluid to be expelled from the injectable fluid chamber barrel 114 via the first passageway 134 and the main passageway 132, in the expulsion configuration. This clockwise rotation of the handle 138 may concurrently fluidly communicatively decouple the main passageway 132 from the second connecting passageway 136.

In the optional closed configuration, the main passageway 132 is not fluidly communicatively coupled with either of the first or the second connecting passageways 134, 136.

While the syringe 110 of Figure 4 includes a stopcock 128 to selectively provide a fluidly communicative path between the communal hub 116, the test chamber, and injectable fluid chamber barrels 112, 114, in other embodiments, the syringe 110 may include other forms of valving mechanisms that are operable to selectively provide one or more fluidly communicative paths.

A lower portion of the communal hub 116 includes an orifice 146 through which a needle 148 is coupled to the communal hub 116. Again, the orifice 146 may be formed by or part of a Luer-Lock connector or coupler. For example, the orifice 146 may be part of a male Luer-Lock portion and the needle 148 may include a female Luer-Lock portion that detachably rotatingly couples with the male Luer-Lock portion. While the embodiment illustrated in Figure 4 includes a Luer-Lock connection, in other embodiments, the communal hub 116 may include a slip-tip, an eccentric tip, or other types of needle adapters to couple the communal hub 116 to the needle 148.

The needle 148 includes a needle hub 149 and a needle shaft 150. The needle shaft 150 includes a beveled end or point or tip 151, and includes a lumen extending therethrough. The needle 148 is fluidly communicatively coupled to the syringe 110 to withdraw or expel fluid when administering to a patient.

With continued reference to Figure 4, the syringe 110 includes a first plunger 152 having a head or plunger seal 154 at a lower end of the first plunger 152. The first plunger 152 is partially received in the test chamber lumen 117 of the test chamber barrel 112. The plunger seal 154 sealingly engages with the interior surface of the test chamber barrel 112 which forms the test chamber lumen 117. The plunger seal 154 may be made from rubber or a polymer, such as an elastomer. The plunger 152 is slideably moveable within the test chamber barrel 112 and includes a thumb rest 153.

The syringe 110 further includes a test indicator 156. The test indicator 156 typically provides a visual indication (*e.g*., appearance of a line, change of color) when contacted by a defined substance. The test indicator 156 is located in the test chamber lumen 117 so as to be contacted by fluid drawn into the test chamber lumen 117 via operation of the first plunger 152. As illustrated in Figure 4, the test indicator 156 may take the form of a cylindrical test indicator 156 coupled to the plunger seal 154. Such may, for example, take the form of a strip of material (*e.g*., woven or nonwoven) or substrate, wrapped around an outer perimeter or periphery of the plunger seal 154. Alternatively, the test indicator 156 may take any other of a large variety of forms. For example, the test indicator 156 may take the form of a rectangular shaped strip of a material with one or more substances (*e.g.*, reagents) that react in a defined manner (*e.g.*, change color) in the presence of defined substances (*e.g*., glucose). The test indicator 156 may additionally or alternatively be coupled to the interior surface of the test chamber barrel 112. Notably, the test indicator 156 should be readily visible from an exterior of the test chamber barrel 112.

The syringe 110 includes a second plunger 162 and a head or plunger seal 164 at a lower end of the plunger 162. The second plunger 162 is partially received in the injectable fluid chamber lumen 119 of the injectable fluid barrel 114. The plunger seal 164 sealingly engages with the interior surface of the injectable fluid barrel 114 which forms the injectable fluid chamber lumen 119. The plunger seal 164 may be made from rubber or a polymer, such as an elastomer. The plunger 162 is slideably moveable within the injectable fluid barrel 114 and includes a thumb rest 163.

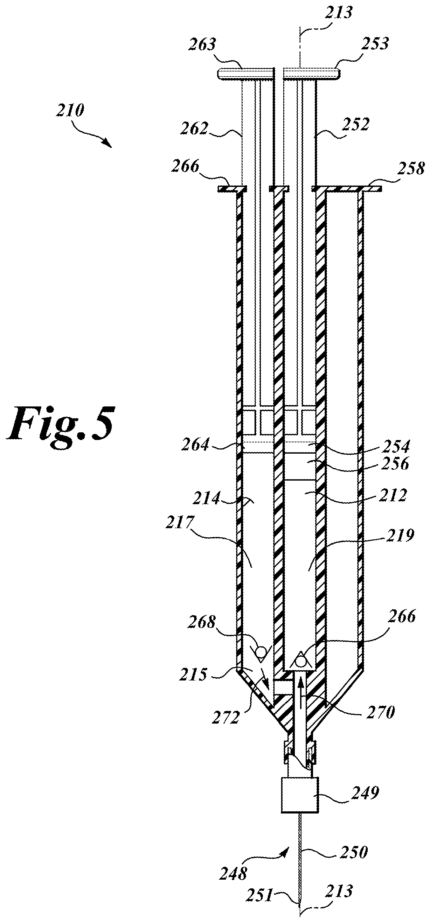

Figure 5 illustrates another example embodiment of a syringe 210. The syringe 210 includes a test chamber barrel 212 and an injectable fluid barrel 214. The chamber barrels 212, 214 each have a respective interior space or lumens 217, 219 and a valving system 215. The test chamber barrel 212 forms an interior barrel that is surrounded by the outer injectable fluid chamber barrel 214. The test chamber barrel 212 and the injectable fluid chamber barrel 214 are both concentric about a central axis 213 of the syringe 210.

An upper end of the test chamber barrel 212 optionally includes a finger flange 258 that extends peripherally around the upper end of the test chamber barrel 212. The finger flange 258 assists a user by providing a gripping surface during use.

An upper end of the injectable fluid chamber barrel 214 optionally includes a finger flange 266 that extends peripherally around the upper end of the injectable fluid chamber barrel 214. The finger flange 266 also assists a user by providing a gripping surface during use.

A lower portion of the syringe 210 includes an orifice through which a needle 248 is coupled to the syringe 210. Again, the orifice may be formed by or part of a LuerLock connector or coupler. For example, the orifice may be part of a male Luer-Lock portion and the needle 248 may include a female Luer-Lock portion that detachably rotatingly couples with the male Luer-Lock portion. Alternatively, the syringe 210 may include a slip-tip, an eccentric tip, or other types of needle adapters to couple to the lower portion of the syringe 210.

The needle 248 includes a needle hub 249 and a needle shaft 250. The needle shaft 250 includes a beveled end or point or tip 251, and includes a lumen extending therethrough. The needle 248 is fluidly communicatively coupled to the syringe 210 to withdraw or expel fluid when administering to a patient.

With continued reference to Figure 5, the syringe 210 includes a first plunger 252 having a head or plunger seal 254 at a lower end of the first plunger 252. The first plunger 252 is partially received in the test chamber lumen 217 of the test chamber barrel 212. The plunger seal 254 sealingly engages with the interior surface of the test chamber barrel 212 which forms the test chamber lumen 217. The plunger seal 254 may be made from rubber or a polymer, such as an elastomer. The plunger 252 is slideably moveable within the test chamber barrel 212 and includes a thumb rest 253.

The syringe 210 further includes a test indicator 256. The test indicator 256 typically provides a visual indication (*e.g.*, appearance of line, change of color) when contacted by a defined substance. The test indicator 256 is located in the test chamber lumen 217 so as to be contacted by fluid drawn into the test chamber lumen 217 via operation of the first plunger 252. As illustrated in Figure 5, the test indicator 256 may take the form of a cylindrical test indicator 256 coupled to the plunger seal 254. Such may, for example, take the form of a strip of material (*e.g.*, woven or nonwoven) or substrate, wrapped around an outer perimeter or periphery of the plunger seal 254. Alternatively, the test indicator 256 may take any other of a large variety of forms. For example, the test indicator 256 may take the form of a rectangular shaped strip of a material with one or more substances (*e.g*., reagents) that react in a defined manner (*e.g*., change color) in the presence of defined substances (*e.g*., glucose). The test indicator 256 may additionally or alternatively be coupled to the interior surface of the test chamber barrel 212. Notably, the test indicator 256 should be readily visible from an exterior of the test chamber barrel 212.

The syringe 210 includes a second plunger 262 and a head or plunger seal 264 at a lower end of the plunger 262. The second plunger 262 is partially received in the injectable fluid chamber lumen 219 of the injectable fluid barrel 214. The plunger seal 264 sealingly engages with the interior surface of the injectable fluid barrel 214 which forms the injectable fluid chamber lumen 219. The plunger seal 264 may be made from rubber or a polymer, such as an elastomer. The plunger 262 is slideably moveable within the injectable fluid barrel 214 and includes a thumb rest 263.

The valving system 215 is fluidly communicatively coupled to the test chamber barrel 212, the injectable fluid chamber barrel 214, and the needle 248. The valving system 215 includes at least a test chamber valve 266 and an injectable fluid chamber valve 268. The test chamber valve 266 and the injectable fluid chamber valve 268 may be one-way valves that allow flow of fluids in one direction. The one-way valves may be duckbill valves, check valves, ball valves, butterfly valves, cross-slit valves, umbrella valves, or the like. By way of example, the illustrated embodiment of Figure 5 includes check valves that are positioned along a lower portion of the syringe 210. The test chamber valve 266 and the injectable fluid chamber valve 268 are operable between an open and a closed position. The test chamber valve 266 and the injectable fluid chamber valve 268 may include a biasing mechanism, such as springs, so that the valves 266, 268 are normally in the closed positions. More particularly, the test chamber valve 266 is configured to allow flow in the direction indicated by arrow 270. The injectable fluid chamber valve 268 is configured to allow flow in the direction indicated by arrow 272.

After a user inserts the syringe 210 at the affected joints, the user may then withdraw fluid from the affected joints. By way of example, distal movement of the first plunger 252 will create negative pressure in the test chamber barrel 212. This pressure will open the test chamber valve 266, fluidly communicatively coupling the needle 248 with the test chamber barrel 212. Fluid will be drawn into the test chamber barrel 212 in the direction 270, while ensuring that the injectable fluid chamber valve 268 remains closed. As the fluid contacts the test indicator 256, the chemical reaction between the fluid and the test indicator 256 may provide an indication of the presence of fluids which are known to be found in joint spaces, as discussed in more detail elsewhere. The user may continue repositioning and withdrawing the fluid until the test indicator 256 indicates appropriate positioning.

Once the user confirms that the tip 251 of the needle 248 is correctly positioned in the joint space, for example, the user may then precisely apply the injectable fluid (*e.g*., an API, such as corticosteroid, hyaluronic acid, or a biologic) by proximally moving the second plunger 262 which will create a positive pressure in the injectable fluid chamber barrel 214. This pressure will open the injectable fluid chamber valve 268, fluidly communicatively coupling the needle 248 with the injectable fluid chamber barrel 214. The injectable fluid may then be expelled in the direction 272, while ensuring that the test chamber valve 266 remains closed.

Although the syringe 210 illustrated in Figure 5 includes two plungers 252, 262, in alternative implementations, a syringe with single plunger may be used. The syringe may further include a vent port positioned at a proximal end of the syringe, which may cause a vacuum to be created during movement of the plunger. In this manner, the single plunger may be used to create the necessary positive or negative pressure differential to withdraw fluid into the interior test chamber barrel and expel fluid from the outer injectable fluid chamber barrel.

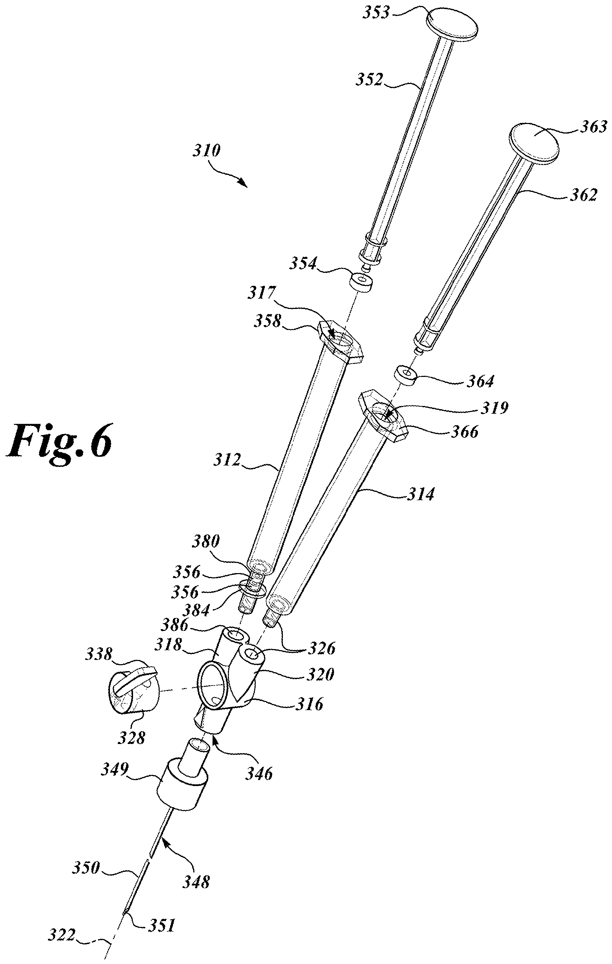

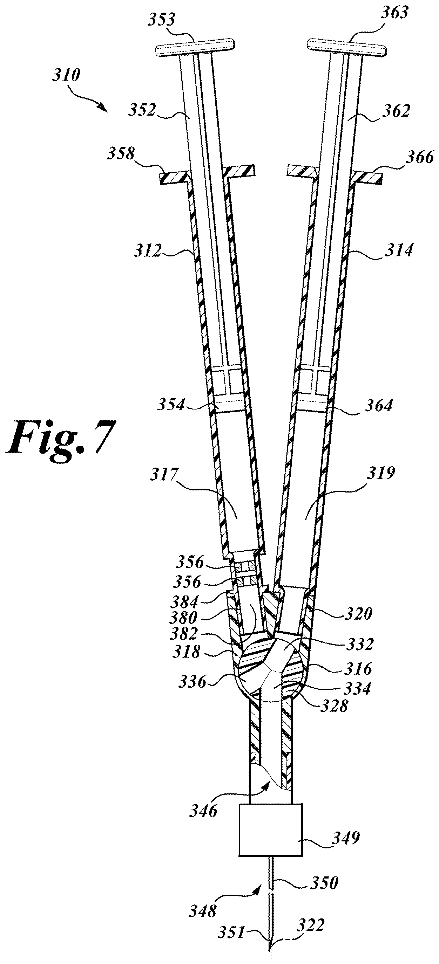

Figures 6 and 7 illustrate another example embodiment of a syringe 310. The syringe 310 includes a test chamber barrel 312 and an injectable fluid chamber barrel 314. Each chamber barrel 312, 314 have a respective interior space or lumens 317, 319. Again, as noted above, the test chamber barrel 312 and the injectable fluid chamber barrel 314 may, in some embodiments, be integrally formed as a single unit, for example, with a web extending therebetween that couples the test chamber barrel 312 to the injectable fluid chamber barrel 314. The syringe 310 may, for example, be formed integrally by plastic injection molding. Alternatively, the test chamber barrel 312 and the injectable fluid chamber barrel 314 may be coupled together, for example via adhesive, hook and loop fastener, other fasteners, etc. The test chamber barrel 312 and the injectable fluid chamber barrel 314 may be formed of transparent or translucent materials, such as clear plastic or glass, to allow a user to view fluids in the interior of the test chamber and injectable fluid chamber barrels 312, 314. In addition, the test chamber and injectable fluid chamber barrels 312, 314 may include graduation markings to allow the user to view the fluid against the graduation markings to assess the volume of fluid in the respective chamber barrels 312, 314.

An upper end of the test chamber barrel 312 optionally includes a finger flange 358 that extends peripherally around the upper end of the test chamber barrel 312. The finger flange 358 assists a user by providing a gripping surface during use.

An upper end of the injectable fluid chamber barrel 314 optionally includes a finger flange 366 that extends peripherally around the upper end of the injectable fluid chamber barrel 314. The finger flange 366 also assists a user by providing a gripping surface during use.

The test chamber barrel 312 and the injectable fluid barrel 314 are coupled to a communal hub 316. The communal hub 316 may, for example, be generally Y-shaped. An upper portion of the Y-shape includes a test chamber adapter 318 and an injectable fluid chamber adapter 320. The test chamber adapter 318 and the injectable fluid chamber adapter 320 are angularly spaced apart from each other about a central reference plane 322. The angular spacing between the test chamber adapter 318 and the injectable fluid chamber adapter 320 may vary between, for example, 10 degrees to 60 degrees.

The test chamber barrel 312 includes an elongated coupler 380, for example in the form of male Luer-Lock portion at a lower end of the test chamber barrel 312. The elongated coupler 380 has a coupler lumen 382. The coupler lumen 382 is configured to receive therein at least one or more test indicators 356. Again, the test indicator 356 typically provides a visual indication (e.g., appearance of a line, change of color) when contacted by a defined substance. In some embodiments, the elongated coupler 380 may include a plurality of test indicators 356, where each test indicator 356 may provide a visual indication for presence of a wide variety of defined substance. For example, one test indicator 356 may provide a visual indication for the presence of one substance, while another test indicator 356 may provide a visual indication for the presence of a different substance.

The test indicators 356 are located in the test coupler lumen 382 so as to be contacted by fluid drawn into the coupler lumen 317 via operation of a first plunger 352. As illustrated in Figures 6 and 7, the test indicators 356 may take the form of a cylindrical test indicator 356 coupled to an interior wall of the elongated coupler 380. The test indicators 356 may, for example, take the form of a strip of material (*e.g*., woven or nonwoven) or substrate, wrapped around an outer perimeter or periphery of the interior wall of the elongated coupler 380. Alternatively, the test indicator 356 may take any other of a large variety of forms. For example, the test indicator 356 may take the form of a rectangular shaped strip of a material with one or more substances (*e.g*., reagents) that react in a defined manner (*e.g*., change color) in the presence of defined substances (*e.g*., glucose). Notably, the test indicator 356 should be readily visible from an exterior of the test chamber barrel 312.

The test chamber barrel 312 also optionally includes an adapter flange 384. The adapter flange 384 surrounds an outer wall of the elongated coupler 380. The adapter flange 384 may, for example, be formed integrally with the test chamber barrel 312. Alternatively, the adapter flange 384 may be separately coupleable to the test chamber barrel 312. In particular, the adapter flange 384 is positioned such that a lower surface of the adapter flange 384 abuts or makes contact with an upper surface of a complementary coupler 386 of the test chamber adapter 318, for example female Luer-Lock portion located in the test chamber adapter 318, when received by the test chamber adapter 318. Further, as illustrated in Figures 6 and 7, the optional adapter flange 384 is located below the test indicator strips 356 so that the test indicator strips 356 may be visible from the exterior of the test chamber barrel 312 when the elongated coupler 380 is received by the test chamber adapter 318.

The injectable fluid chamber 314 also includes a coupling adapter 326, for example, in the form of female and male Luer-Lock portions, which couple the injectable fluid chamber barrel 314 to the communal hub 316. Male coupler, for example, in the form of male Luer-Lock portion is located at a lower end of the injectable fluid chamber barrel 314. Female coupler, for example, in the form of female Luer-Lock portion, is located at the injectable fluid chamber adapter 320.

The elongated coupler 380 of the test chamber barrel 312 and the male Luer-Lock portion of the injectable fluid chamber 314 are physically detachably coupleable to corresponding female Luer-Lock portions. In this manner, the test chamber barrel 312 and/or the injectable fluid chamber barrel 314 may each be selectively detachable from the syringe 310.

A central portion of the communal hub 316 is substantially cylindrical and hollow, and includes a valve, for example, a stopcock 328, coupled to the hollow portion of the communal hub 316. The stopcock 328 includes at least a two-way flow system. The two-way flow system includes a main passageway 332, a first connecting passageway 334, and a second connecting passageway 336. The stopcock 328 is operable to selectively fluidly communicatively couple the main passageway 332 to the first and second connecting passageways 334, 336. By way of example, the stopcock 328 includes a handle 338 which is rotatable such that the stopcock 328 is selectively operable in one of the at least two configurations, namely withdrawal and expulsion. In some implementations, the stopcock 328 is selectively operable in one of three configurations, namely withdrawal, expulsion, and closed (*i.e.,* the main passageway 332 is not fluidly communicatively connected to either one of the first or second connecting passageways 334, 336).

By way of example, rotating the handle 338 counterclockwise such that a withdrawal arrow is aligned with a main arrow, fluidly communicatively couples the main passageway 332 with the second connecting passageway 336, allowing a fluid to be drawn to the test chamber barrel 312 from the main passageway 332, in the withdrawal configuration. This counterclockwise rotation of the handle 338 may concurrently fluidly communicatively decouple the main passageway 332 from the first connecting passageway 334. Conversely, rotating the handle 338 in a clockwise direction such that an expulsion arrow is aligned with the main arrow, fluidly communicatively couples the main passageway 332 with the first connecting passageway 334, allowing fluid to be expelled from the injectable fluid chamber barrel 314 via the first passageway 334 and the main passageway 332, in the expulsion configuration. This clockwise rotation of the handle 338 may concurrently fluidly communicatively decouple the main passageway 332 from the second connecting passageway 336.

In the optional closed configuration, the main passageway 332 is not fluidly communicatively coupled with either of the first or the second connecting passageways 334, 336.

While the syringe 310 of Figures 6 and 7 includes a stopcock 328 to selectively provide a fluidly communicative path between the communal hub 316, the test chamber, and injectable fluid chamber barrels 312, 314, in other embodiments, the syringe 310 may include other forms of valving mechanisms that are operable to selectively provide one or more fluidly communicative paths.

A lower portion of the communal hub 316 includes an orifice 346 through which a needle 348 is coupled to the communal hub 316. Again, the orifice 346 may be formed by or part of a Luer-Lock connector or coupler. For example, the orifice 346 may be part of a male Luer-Lock portion and the needle 348 may include a female Luer-Lock portion that detachably rotatingly couples with the male Luer-Lock portion, or vice versa. While the embodiment illustrated in Figures 6 and 7 include a Luer-Lock connection, in other embodiments, the communal hub 316 may include a slip-tip, an eccentric tip, or other types of needle adapters to couple the communal hub 316 to the needle 348.

The needle 348 includes a needle hub 349 and a needle shaft 350. The needle shaft 350 includes a beveled end or point or tip 351, and includes a lumen extending therethrough. The needle 348 is fluidly communicatively coupled to the syringe 310 to withdraw or expel fluid when administering to a patient.

With continued reference to Figures 6 and 7, the syringe 310 includes a first plunger 352 having a head or plunger seal 354 at a lower end of the first plunger 352. The first plunger 352 is partially received in the test chamber lumen 317 of the test chamber barrel 312. The plunger seal 354 sealingly engages with the interior surface of the test chamber barrel 312 which forms the test chamber lumen 317. The plunger seal 354 may be made from rubber or a polymer, such as an elastomer. The plunger 352 is slideably moveable within the test chamber barrel 312 and includes a thumb rest 353.

The syringe 310 includes a second plunger 362 and a head or plunger seal 364 at a lower end of the plunger 362. The second plunger 362 is partially received in the injectable fluid chamber lumen 319 of the injectable fluid barrel 314. The plunger seal 364 sealingly engages with the interior surface of the injectable fluid barrel 314 which forms the injectable fluid chamber lumen 319. The plunger seal 364 may be made from rubber or a polymer, such as an elastomer. The plunger 362 is slideably moveable within the injectable fluid barrel 314 and includes a thumb rest 363.

The various embodiments described above can be combined to provide further embodiments. For example, in some implementations, the test chamber barrel may advantageously be separable from the syringe. The test chamber barrel may be removed and sent to a laboratory for testing of the withdrawn specimens. Alternatively, the syringe may be disposable. The syringe or components of the syringe may be disposed after single use.

Further, various implementations may employ other indicator strips, for example indicator strips that indicate a presence or even a level or concentration of other body fluids, such as protein or hyaluronic acids. Indicator strips may, for example, perform chromatographic assays. Some such indicator strips are commonly referred to as assay test strips or immunochromatographic strips. One type of assay strip is commonly referred to as chromatographic lateral flow strips or lateral flow strips. Other types of assay strips include western blots, southern blots, electrophoresis gels, dot blots, etc. Assay strips may be used for both qualitative and semi-quantitative assays, which typically employ visual detection.

Assay strips typically take the form of a substrate comprising a matrix of material through which a fluid test sample, which may or may not contain an analyte that is being tested for, can flow or be absorbed. In use, a liquid test sample is applied to a portion of the assay strip to detect a presence or absence of an analyte. In the case of lateral flow strips, the fluid to be tested and analyte suspended or dissolved therein flow from the application zone to a detection zone, for example via capillary action. The test fluid typically flows laterally though the matrix. The appearance or absence of a visible signal (*e.g*., test results signal line) at the detection zone reveals the presence or absence of the analyte. Assay strips typically visually display two parallel lines, known as capture or signal lines. One of the lines indicates that test strip performance has not been compromised. The second line becomes visible only when the sample contains an amount of analyte in excess of a minimum or threshold concentration. In such assay strips, the capture or signal lines consist of immobilized capture reagents or receptors which are pre-applied to the matrix during manufacture.

Lateral flow type assay strips may include a binding partner that immunospecifically binds the analyte to be detected and which bears a detectable label. The binding may be competitive binding or non-competitive. Competitive assays are particularly suited to detect smaller molecules, such as drugs and drug metabolites. Non-competitive immunoassays are primarily used for detection of large molecules such as proteins, large hormones, or molecules which have multiple binding sites. The detection zone may include a substrate for a label capable of providing a colored response in the presence of the label. The assay strip may also contain a zone in which analyte is immobilized so that labeled binding partner which is not combined due to an absence of analyte in the sample will be captured and prevented from reaching the detection zone. Multi-zone assay strips are also possible, which may include a detection zone that contains an immobilized form of a binding substance for a labeled reagent. The labeled reagent bears a detectable chemical group having a detectable physical property so that it does not require a chemical reaction with another substance. Examples of such include colored species, fluorescers, phosphorescent molecules, radioisotopes and electro-active moieties.

Moreover, the various components described herein may advantageously be provided as a kit. The kit may, for example, include a communal hub with a test indicator. The test indicator may include a material with one or more substances (*e.g*., reagents) that react in a defined manner (*e.g*., change color) in the presence of defined substances. The communal hub may include a valving mechanism that is operable to selectively provide one or more fluidly communicative paths. The communal hub may include adapters such that the communal hub is coupleable to a needle and chamber barrels. The kit may also include test chamber and injectable fluid chamber barrels to precisely confirm and administer injectable fluids to the affected areas. Alternatively, the kit may only include a communal hub, test indicator, and a test chamber barrel. The injectable fluid barrel may be supplied by the user. The kit may also include injectable fluids or medicant(s) that are being administered at the affected areas and needles. The kit may also include a set of instructions for effective use of the syringe.

## Claims

1. A syringe (10) for intra-articular injection, comprising:
a hub (16) having an orifice (46);
a first barrel (12) having an interior surface that forms a first barrel lumen (17);
a first plunger (52) having a head (54), the head of the first plunger (54) slideably received in the first barrel lumen (17) for movement therein, the head (54) of the first plunger in sealing engagement with the interior surface of the first barrel (12);
a second barrel (14) having an interior surface that forms a second barrel lumen (19);
a second plunger (62) having a head (64), the head of the second plunger (64) slideably received in the second barrel lumen (19) for movement therein, the head of the second plunger (64) in sealing engagement with the interior surface of the second barrel (17);
a valve (28) operable in a first condition to open a fluidly communicative path between the orifice of the hub (16) and the first barrel lumen (17) and to close a fluidly communicative path between the orifice of the hub (16) and the second barrel lumen (19), and the valve operable in a second condition to open the fluidly communicative path between the orifice of the hub (16) and the second barrel lumen (19) and to close the fluidly communicative path between the orifice of the hub (16) and the first barrel lumen (17);
a needle (48) that is coupled to the hub (16), and insertable into a joint; and
a test indicator (56) that is optically responsive to the presence of glucose in a bodily fluid, the test indicator positioned to be exposed to bodily fluid drawn into the first barrel lumen through the needle and visible from an exterior of the first barrel (12);
wherein a defined visual indication by the test indicator (56) indicates appropriate positioning of the needle (48) in the joint, and wherein the needle may be repositioned if the test indicator (56) does not indicate appropriate positioning.

2. The syringe (10) of claim 1 wherein the test indicator (56) takes the form of a test strip coupled to the interior surface of the first barrel (12).

3. The syringe (10) of claim 1, further comprising:
an injectable fluid loaded in the second barrel lumen (19) for delivery from the syringe.

4. The syringe of claim 3 wherein the injectable fluid comprises at least one of a corticosteroid, hyaluronic acid or a biologic loaded in the second barrel lumen (19).

5. The syringe of any one of the preceding claims wherein the second barrel (14) is selectively detachable from at least the first barrel (12).

6. The syringe of any one of the preceding claims wherein the first (12) and the second (14) barrels are arranged either in a side-by-side tandem configuration or in a concentric configuration.

7. The syringe of any one of the preceding claims wherein the first barrel includes an elongated coupler that is selectively detachably received in the hub (16).

8. The syringe of any one of the preceding claims, further comprising:
a plurality of test indicators (56), at least one of the plurality of test indicators positioned in the elongated coupler.

## Patentansprüche

1. Spritze (10) zur intraartikulären Injektion, die Folgendes umfasst:
einen Verteiler (16), der eine Öffnung (46) aufweist;
einen ersten Spritzenzylinder (12), der eine Innenfläche aufweist, die ein erstes Spritzenzylinder-Lumen (17) ausbildet;
einen ersten Kolben (52), der einen Kopf (54) aufweist, wobei der Kopf des ersten Kolbens (54) im ersten Spritzenzylinder-Lumen (17) zur Bewegung in diesem verschiebbar aufgenommen ist, wobei der Kopf (54) des ersten Kolbens in dichtendem Eingriff mit der Innenfläche des ersten Spritzenzylinders (12) steht;
einen zweiten Spritzenzylinder (14), der eine Innenfläche aufweist, die ein zweites Spritzenzylinder-Lumen (19) ausbildet;
einen zweiten Kolben (62), der einen Kopf (64) aufweist, wobei der Kopf des zweiten Kolbens (64) im zweiten Spritzenzylinder-Lumen (19) zur Bewegung in diesem verschiebbar aufgenommen ist, wobei der Kopf des zweiten Kolbens (64) in dichtendem Eingriff mit der Innenfläche des zweiten Spritzenzylinders (17) steht;
ein Ventil (28), das in einem ersten Zustand betätigbar ist, um einen Fluidkommunikationspfad zwischen der Öffnung des Verteilers (16) und dem ersten Spritzenzylinder-Lumen (17) zu öffnen und einen Fluidkommunikationspfad zwischen der Öffnung des Verteilers (16) und dem zweiten Spritzenzylinder-Lumen (19) zu schließen, und wobei das Ventil in einem zweiten Zustand betätigbar ist, um den Fluidkommunikationspfad zwischen der Öffnung des Verteilers (16) und dem zweiten Spritzenzylinder-Lumen (19) zu öffnen und den Fluidkommunikationspfad zwischen der Öffnung des Verteilers (16) und dem ersten Spritzenzylinder-Lumen (17) zu schließen;
eine Nadel (48), die mit dem Verteiler (16) gekoppelt und in ein Verbindungsstück einbringbar ist; und
einen Testindikator (56), der optisch auf das Vorliegen von Glukose in einer Körperflüssigkeit reagiert, wobei der Testindikator angeordnet ist, um einer Körperflüssigkeit, die über die Nadel in das erste Spritzenzylinder-Lumen gezogen wird, ausgesetzt zu sein und von außerhalb des ersten Spritzenzylinders (12) sichtbar zu sein;
wobei eine definierte visuelle Anzeige durch den Testindikator (56) die ordnungsgemäße Anordnung der Nadel (48) im Verbindungsstück anzeigt und wobei die Nadel neu angeordnet werden kann, wenn der Testindikator (56) eine nicht ordnungsgemäße Anordnung anzeigt.

2. Spritze (10) nach Anspruch 1, wobei der Testindikator (56) die Form eines Teststreifens, der mit der Innenfläche des ersten Spritzenzylinders (12) gekoppelt ist, aufweist.

3. Spritze (10) nach Anspruch 1, die ferner Folgendes umfasst:
ein injizierbares Fluid, das zur Zufuhr aus der Spritze in das zweite Spritzenzylinder-Lumen (19) gefüllt ist.

4. Spritze nach Anspruch 3, wobei das injizierbare Fluid zumindest eines aus einem Corticosteroid, Hyaluronsäure oder einer in das zweite Spritzenzylinder-Lumen (19) gefüllten biologischen Substanz umfasst.

5. Spritze nach einem der vorhergehenden Ansprüche, wobei der zweite Spritzenzylinder (14) selektiv zumindest vom ersten Spritzenzylinder (12) lösbar ist.

6. Spritze nach einem der vorhergehenden Ansprüche, wobei der erste (12) und der zweite (14) Spritzenzylinder entweder in einer Seite-an-Seite-Tandemkonfiguration oder in einer konzentrischen Konfiguration angeordnet sind.

7. Spritze nach einem der vorhergehenden Ansprüche, wobei der erste Spritzenzylinder ein längliches Kopplungselement umfasst, das selektiv lösbar im Verteiler (16) aufgenommen ist.

8. Spritze nach einem der vorhergehenden Ansprüche, die ferner Folgendes umfasst:
eine Vielzahl von Testindikatoren (56), wobei zumindest einer der Vielzahl von Testindikatoren im länglichen Kopplungselement angeordnet ist.

## Revendications

1. Seringue (10) pour injection intra-articulaire, comprenant :
un raccord (16) ayant un orifice (46) ;
un premier cylindre (12) ayant une surface intérieure qui forme une première lumière de cylindre (17) ;
un premier piston (52) ayant une tête (54), la tête du premier piston (54) étant reçue de manière coulissante dans la première lumière de cylindre (17) pour s'y déplacer, la tête (54) du premier piston étant en prise de manière étanche avec surface intérieure du premier cylindre (12) ;
un second cylindre (14) ayant une surface intérieure qui forme une seconde lumière de cylindre (19) ;
un second piston (62) ayant une tête (64), la tête du second piston (64) étant reçue de manière coulissante dans la seconde lumière de cylindre (19) pour s'y déplacer, la tête du second piston (64) étant en prise de manière étanche avec la surface intérieure du second cylindre (17) ;
une valve (28) opérationnelle dans un premier état pour ouvrir un trajet de communication fluidique entre l'orifice du raccord (16) et la première lumière de cylindre (17) et pour fermer un trajet de communication fluidique entre l'orifice du raccord (16) et la seconde lumière de cylindre (19), et la vanne étant opérationnelle dans un second état pour ouvrir le trajet de communication fluidique entre l'orifice du raccord (16) et la second lumière de cylindre (19) et pour fermer le trajet de communication fluidique entre l'orifice du raccord (16) et la première lumière de cylindre (17) ;
une aiguille (48) qui est couplée au raccord (16), et pouvant être insérée dans un joint ; et
un indicateur de test (56) qui réagit optiquement à la présence de glucose dans un fluide corporel, l'indicateur de test étant positionné pour être exposé à un fluide corporel aspiré dans la première lumière de cylindre à travers l'aiguille et visible depuis un extérieur du premier cylindre (12)
dans lequel une indication visuelle définie par l'indicateur de test (56) indique un positionnement approprié de l'aiguille (48) dans le joint, et dans lequel l'aiguille peut être repositionnée si l'indicateur de test (56) n'indique pas un positionnement approprié.

2. Seringue (10) selon la revendication 1, dans laquelle l'indicateur de test (56) se présente sous la forme d'une bandelette de test couplée à la surface intérieure du premier cylindre (12).

3. Seringue (10) selon la revendication 1, comprenant en outre :
un fluide injectable chargé dans la seconde lumière de cylindre (19) pour être délivré à partir de la seringue.

4. Seringue selon la revendication 3, dans laquelle le fluide injectable comprend au moins l'un d'un corticostéroïde, de l'acide hyaluronique ou d'un agent biologique chargé dans la seconde lumière de cylindre (19).

5. Seringue selon l'une quelconque des revendications précédentes, dans laquelle le second cylindre (14) peut être détaché sélectivement d'au moins le premier cylindre (12).

6. Seringue selon l'une quelconque des revendications précédentes, dans laquelle les premier (12) et second (14) cylindres sont disposés soit dans une configuration en tandem côte à côte, soit dans une configuration concentrique.

7. Seringue selon l'une quelconque des revendications précédentes, dans laquelle le premier cylindre comprend un coupleur allongé qui est reçu dans le raccord (16) en pouvant être détaché sélectivement.

8. Seringue selon l'une quelconque des revendications précédentes, comprenant en outre :
une pluralité d'indicateurs de test (56), au moins l'un de la pluralité d'indicateurs de test étant positionné dans le coupleur allongé.
